# EUROPÄISCHE PATENTSCHRIFT

(11) **EP 1 834 574 B1**
(45) Veröffentlichungstag und Bekanntmachung des Hinweises auf die Patenterteilung: **29.10.2008**
(21) Anmeldenummer: 07002978.0
(22) Anmeldetag: 13.02.2007
(51) Int. Cl.: A61B 1/002, G02B 13/00

(54) **Endoskoprohr mit Bildumkehrsystem**
Endoscope tube with reverse image system
Gaine d'endoscope dotée d'un système de retournement d'image

(30) Priorität: 16.03.2006 DE 102006012563
(43) Veröffentlichungstag der Anmeldung: 19.09.2007
(73) Patentinhaber: Karl Storz GmbH & Co. KG, 78532 Tuttlingen (DE)
(72) Erfinder: Lei, Fang, 78591 Durchhausen (DE)
(74) Vertreter: Stamer, Harald

(56) Entgegenhaltungen:
- DE-A1- 3 113 110
- DE-A1- 3 431 631
- DE-A1- 3 839 364

## Beschreibung

Die Erfindung betrifft ein Endoskoprohr mit mindestens einem aus zwei Stablinsen bestehenden Bildumkehrsystem entsprechend den Merkmalen des Oberbegriffs des Anspruchs 1.

Ein solches System ist aus DE 38 39 364 C2 bekannt. Jede der Stablinsen weist an einem ersten Stabende einen Randzylinder auf, dessen Durchmesser an den Innendurchmesser des Endoskoprohres angepaßt ist. Der Randzylinder hat eine Länge von etwa zwei Drittel der Gesamtlänge der Stablinse und dient der Lagerung der Stablinse in dem Endoskoprohr. Das zweite Stabende weist ebenfalls einen Randzylinder auf, dessen Durchmesser aber geringer als der Innendurchmesser des Endoskoprohres ist. Der sprunghafte Übergang von einem dickeren zu einem dünneren Stablinsenteil erhöht die Bruchgefahr an dieser Stelle bei der Bearbeitung und beim Einsetzen in das Endoskoprohr.

Die Endfläche des dickeren Randzylinders ist konvex. An die Endfläche des dünneren Randzylinders ist eine Korrekturlinse angekittet. Die konvexen Endflächen weisen zu einer Zwischenbildebene. Die Korrekturlinsen schließen eine Blendenebene ein.

Es ist bekannt, daß Stablinsen im Bereich von Kittflächen bei einer Biegebeanspruchung des Endoskoprohres einer erhöhten Bruchgefahr ausgesetzt sind. Die Verringerung des Stabdurchmessers in diesem Bereich soll eine Auswirkung der Biegebeanspruchung auf den Kittbereich vermeiden. Zur Ausbildung des Blendenraumes ist es jedoch erforderlich, daß zwischen die beiden Stablinsen ein Abstandhalter eingefügt wird. Dieser ist als Röhrchen ausgebildet, das einerseits am Endoskoprohr anliegt und sich andererseits in axialer Richtung an den Korrekturlinsen abstützt. Bei einer Biegebeanspruchung des Endoskoprohres läßt es sich daher nicht vermeiden, daß über das Abstandsröhrchen radial unterschiedliche axiale Druckkräfte auf die angekitteten Korrekturlinsen wirken. Diese Druckkräfte können zu einer Beschädigung der Kittfläche oder der Endfläche und damit zur Verschlechterung der optischen Abbildungsqualität führen.

Eine Biegebeanspruchung des Endoskoprohres wirkt unmittelbar auch auf den relativ langen dickeren Randzylinder und kann zum Brechen der Stablinse führen.

Aus DE 31 13 110 A1 sind Stablinsen mit zwei zylindrischen Endstücken bekannt, die am Endoskoprohr anliegen sollen. Zur Verminderung der Bruchempfindlichkeit soll der Stabdurchmesser in der Mitte der Längserstreckung kleiner als an den Enden sein. Die Verringerung zwischen den Endstücken kann einen radialen Verlauf haben. Zwischen mehreren solcher Stablinsen können Abstandsrohre angeordnet sein.

Aus DE 34 31 631 A1 sind ringförmige Halterungsmittel für gleichförmig zylindrische Stablinsen bekannt, die die Stablinsen insgesamt im Radialabstand zur Optikrohr-Innenfläche halten. Die Halterungen sind vorzugsweise als Axialabstandsrohre mit Endkragen ausgebildet.

Der Erfindung lag daher die Aufgabe zugrunde, die Stablinsen so auszubilden, daß ihre Bruchempfindlichkeit bei der Anordnung in einem Endoskoprohr minimiert wird und einen daran gepaßten Abstandhalter anzugeben.

Diese Aufgabe wird bei einem Endoskoprohr mit Bildumkehrsystem der eingangs genannten Art erfindungsgemäß durch die kennzeichnenden Merkmale des Anspruchs 1 gelöst. Vorteilhafte Ausgestaltungen und Weiterbildungen ergeben sich aus den Merkmalen der Unteransprüche.

Der durch die Verjüngung der Stabenden an dieser Stelle erreichte größere Abstand zum Endoskoprohr kann zur Ausbildung des Abstandhalters als elastischer Stützkörper für die verjüngten Stabenden genutzt werden.

Das sich kontinuierlich verjüngende, insbesondere konusförmige Stabende der Stablinsen ermöglicht eine in axialer Richtung fixierte Lagerung dieses Stabendes über seine Mantelfläche ohne Berührung zum Endoskoprohr. Bei einer Kegelform des Stabendes kann das Lager in besonders einfacher Weise der Mantelfläche angepaßt werden. Durch das Verhältnis der Durchmesser der Stabenden zu der Stablänge ergeben sich relativ große Kegelwinkel, die die Fixierung des Lagers in axialer Richtung unterstützen. Der relativ kurze dickere Randzylinder bietet wenig Angriffsfläche für Biegekräfte am Endoskoprohr.

Für die Lagerung der kegelförmigen Stabenden ist insbesondere ein Abstandhalter geeignet, der beidseitig eine zumindest kurze trichterförmige Aufnahme enthält. Der Trichter ist zum Blendenraum offen. Durch die Länge des Abstandhalters sowie den Öffnungswinkel und Öffnungsdurchmesser des Trichters kann die Positionierung der beiden Stabenden so eingestellt werden, daß die jeweiligen Kittflächen oder Endflächen der Stabenden frei im Blendenraum liegen und der Abstand der Linsenelemente zur Blendenebene einem vorgegebenen Wert entspricht.

Der Abstandhalter wird vorzugsweise aus einem elastischen Material gefertigt, das durch Stöße erzeugte Erschütterungen und auch stärkere Biegungen am Endoskoprohr noch auffangen kann. Zusätzlich kann der Abstandhalter so ausgebildet werden, daß er sich radial federnd am Endoskoprohr abstützt. Der Abstandhalter kann einstückig als Spritzgußteil ausgeführt sein. Es ist jedoch auch möglich, den Abstandhalter aus mehreren in axialer Richtung geteilten Segmenten zu fertigen.

Durch eine in radialer Richtung elastische und in axialer Richtung starre Ausbildung des Abstandhalters mit an den Außenrändern der Strahlendurchtrittsöffnung erweiterten Aufnahmen für die verjüngten Stabenden ist auch eine schonende stirnseitige Aufnahme der Stabenden möglich.

Ein Ausführungsbeispiel für ein erfindungsgemäßes Endoskoprohr mit Bildumkehrsystem ist in der Zeichnung schematisch dargestellt und wird nachfolgend anhand der Figuren näher beschrieben. Dabei zeigen
Fig.1 ein bekanntes Endoskop mit starrem Einführungsteil,
Fig.2 einen Ausschnitt des Einführungsteils im Querschnitt,
Fig.2a,b verschiedene Ausführungen eines Abstandhalters,
Fig.3 den Querschnitt eines weiteren Ausführungsbeispiels und
Fig.4 einen scheibenförmigen Abstandhalter mit stirnseitiger Anlage der nicht Teil der Erfindung ist.

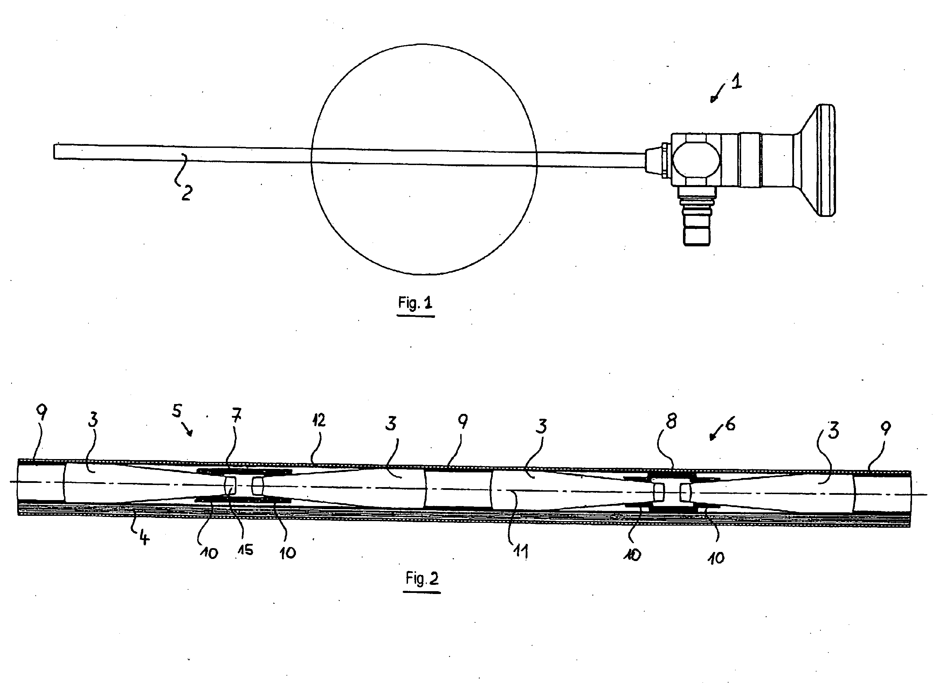

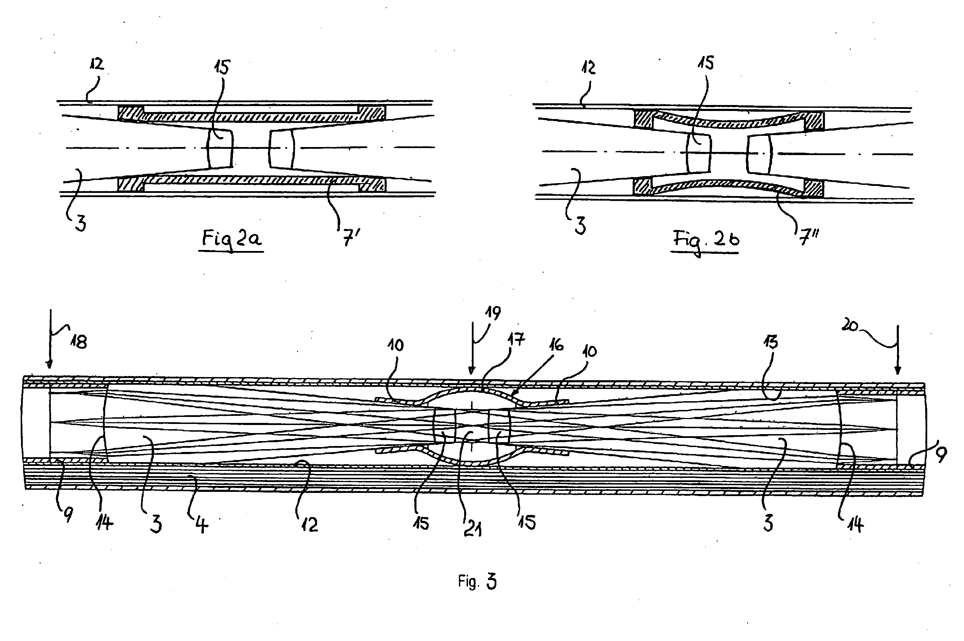

Fig.1 zeigt ein bekanntes Endoskop 1 mit starrem Einführungsteil 2. Ein mit einem Kreis gekennzeichneter Ausschnitt aus dem Einführungsteil ist in Fig.2 im Querschnitt schematisch dargestellt. Das Einführungsteil 2 enthält im allgemeinen separate Rohre zur Lagerung der Stablinsen 3 und eines Beleuchtungsfaserbündels 4, wie in Fig.3 genauer dargestellt ist.

In Fig.2 sind zwei Bildumkehrsysteme 5, 6 dargestellt. Jedes der Bildumkehrsysteme 5, 6 besteht aus jeweils zwei kegelförmigen Stablinsen 3, die im Bereich der dünneren Stabenden von einem Abstandhalter 7, 8 zusammengehalten und zueinander ausgerichtet werden. Betrachtet man die beiden an den dünneren Stabenden einander gegenüberliegenden Stablinsen, so können diese jeweils als eine Hälfte einer knochenförmigen Stablinse gesehen werden, die durch den Abstandhalter zusammengehalten werden. Die beiden Bildumkehrsysteme 5, 6 sind gegeneinander durch ein Abstandsröhrchen 9 beabstandet.

Ein erster Abstandhalter 7 enthält in axialer Richtung beidseitig je eine trichterförmige Aufnahme 10, die das konusförmige dünnere Stabende der Stablinse 3 umschließt. Der Abstandhalter 7 ist frei tragend mit den Stablinsen 3 verbunden. Wie Fig. 2a zeigt, kann der Abstandhalter 7' im Bereich der trichterförmigen Aufnahme 10 auch mit einem aufgewulsteten Rand zur Abstützung an der Rohrinnenwand versehen sein. Anstelle eines zylindrischen Abstandteiles kann dieses auch als taillierter Rotationskörper 7" ausgebildet sein der eine verbesserte Steifigkeit gegenüber Biegekräften aufweist.

Ein zweiter Abstandhalter 8 enthält ebenfalls beidseitig eine trichterförmige Aufnahme 10. Im mittleren Bereich stützt er sich jedoch an der Rohrinnenwand ab. Auf diese Weise wird zusätzlich eine bessere Lastverteilung für die Stabenden und bessere Zentrierung der optischen Achsen11 der Stablinsen 3 erreicht. Die Abstützung kann elastisch ausgebildet sein.

In Fig.3 ist ein vergrößerter Ausschnitt aus dem Einführungsteil 2 mit einem Bildumkehrsystem 6 im Detail dargestellt. In einem separaten Endoskoprohr 12 sind die beiden Stablinsen 3 mit ihren Randzylindern 13 gelagert. Die Stablinsen 3 weisen an diesem dickeren Ende konvexe Frontflächen 14 auf. An die dünneren Enden der Stablinsen 3 sind Linsenelemente 15 angekittet.

Der weitere Abstandhalter 16 besitzt einen mittleren Teil, der als bauchiger Rotationskörper 17 ausgebildet ist. Beidseitig sind trichterförmige Aufnahmen 10 angeformt, deren Öffnungswinkel an die kegelförmige Verjüngung der Stablinsen 3 angepaßt ist. Der rotationsförmig ballige Mittelteil des Abstandhalters 16 stützt sich am Endoskoprohr 12 federnd ab. Der innere Öffnungsdurchmesser des trichterförmigen Teiles ist so groß, daß die Kittfläche für das Linsenelement 15 im vollständig eingeschobenen Zustand der Stablinse 3 außerhalb der Aufnahme 10 liegt. Der Abstandhalter 16 kann aus Metall oder Kunststoff gefertigt und mit den kegelförmigen Teilen der Stablinsen 3 verklebt sein.

Die geometrischen Abmessungen der Stablinsen 3 sind so gewählt, daß sich im Bereich der trichterförmigen Aufnahme 10 eine für eine sichere axiale Fixierung ausreichende Kegelform am dünneren Ende der Stablinse 3 ergibt. Die Länge des Randzylinders 13 soll kleiner bis gleich einem Drittel der Gesamtlänge der Stablinse 3 sein, damit genügend Raum für die Biegung des Endoskoprohres zur Verfügung steht. Die Länge des Randzylinders 13 soll aber größer als der halbe Durchmesser der Stablinse 3 an dieser Stelle sein, damit genügend Fläche zum Stützen der Stablinse 3 entsteht. Wenn der Durchmesser des Randzylinders 13 unter diesen Bedingungen etwa einem Fünftel der Stablänge und der Durchmesser der Endfläche des anderen Stabendes etwa einem Zehntel der Stablänge entspricht, ergibt sich ein Kegelwinkel, der eine sichere Anlage in der trichterförmigen Aufnahme 10 auch ohne zusätzliche Verklebung gewährleistet.

Aus dem ebenfalls dargestellten Verlauf der Abbildungstrahlenbündel von einer ersten Zwischenbildebene 18 über die Blendenebene 19 zu einer zweiten Zwischenbildebene 20 läßt sich erkennen, daß das System eine zu herkömmlichen Stablinsen-Anordnungen vergleichbare numerische Apertur besitzt. Diese wird bei den konstruktionsbedingt kurzen Stablängen durch eine entsprechende Einengung des effektiven Durchmessers im Blendenraum 21 erreicht. Diese Einengung schafft andererseits den Raum für die Einfügung der erfindungsgemäßen Abstandhalter bzw. Stützkörper.

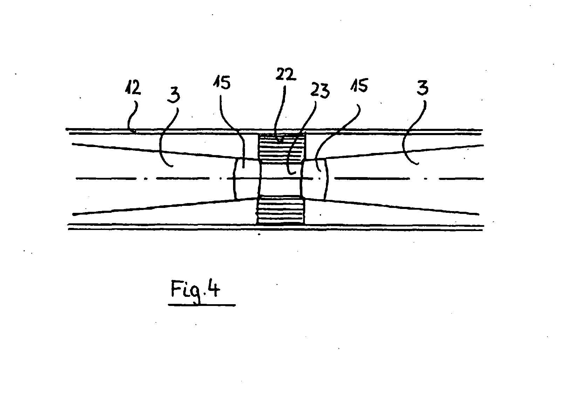

Fig.4 zeigt einen scheibenförmigen Abstandhalter mit einer Strahlendurchgangsöffnung 23 für eine stirnseitige Aufnahme der verjüngten Stabenden. Die waagerechten Striche im Querschnitt des scheibenförmigen Körpers 22 sollen die Steifigkeit in axialer Richtung und der Abstand der Striche die Elastizität in radialer Richtung darstellen. Bei einer Biegebeanspruchung des Endoskoprohres wirken nahezu keine Scherkräfte auf die angekitteten Linsenelemente 15 an den Stabenden, da diese über den relativ dicken elastischen Scheibenring abgeleitet werden. Wenn der Scheibenkörper aus Kunststoff gefertigt ist, entstehen auch keine Reibungskräfte, die zu einer Beschädigung der Oberfläche führen können. Die Anlage am Abstandhalter 22 erfolgt nur in einem kleinen Randbereich der Strahlendurchtrittsöffnung 23. Besonders vorteilhaft ist diese Form des Abstandhalters 22 bei verjüngten Stabenden ohne aufgekittete Linsenelemente, wie dies z.B. bei dem bereits erwähnten Zusammenfügen von zwei halben knochenförmigen Stablinsen der Fall ist.

### Bezugszeichenliste

- 1: Endoskop
- 2: Einführungsteil
- 3: Stablinsen
- 4: Beleuchtungsfaserbündel
- 5,6: Bildumkehrsystem
- 7,7',7": erster Abstandhalter
- 8: zweiter Abstandhalter
- 9: Abstandsröhrchen
- 10: trichterförmige Aufnahme
- 11: optische Achse
- 12: Endoskoprohr
- 13: Randzylinder
- 14: konvexe Frontfläche
- 15: Linsenelement
- 16: weiterer Abstandhalter
- 17: bauchiger Rotationskörper
- 18: erste Zwischenbildebene
- 19: Blendenebene
- 20: zweite Zwischenbildebene
- 21: Blendenraum
- 22: scheibenförmiger Körper
- 23: Strahlendurchgangsöffnung

## Patentansprüche

1. Endoskoprohr mit mindestens einem aus zwei Stablinsen (3) bestehenden Bildumkehrsystem (5, 6), bei dem die zu einer Zwischenbildebene (18, 20) weisenden ersten Stabenden einen Randzylinder (13) aufweisen, der in dem Endoskoprohr (12) gelagert ist, bei dem die zu einer Blendenebene (19) symmetrisch liegenden zweiten Stabenden einen Durchmesser aufweisen, der kleiner als der Innendurchmesser des Endoskoprohres (12) ist, wobei die den ersten Stabenden zugeordneten Endflächen konvex ausgebildet sind, die den zweiten Stabenden zugeordneten Endflächen mit einem Linsenelement (15) verkittet sein können und zwischen den beiden Stablinsen (3) ein den Blendenraum (21) definierender Abstandhalter (7, 7', 7"; 8; 16) eingefügt ist, **dadurch gekennzeichnet, daß** der Stabdurchmesser jeweils vom Randzylinder (13) zum zweiten Stabende hin kontinuierlich über die gesamte Stablänge abnimmt und der Abstandhalter (7, 7', 7"; 8; 16) in axialer Richtung beidseitig mit einer zum Blendenraum (21) offenen an die Abnahme des Stabdurchmessers angepaßten, trichterförmig ausgebildeten Aufnahme (10) für die zweiten Stabenden ausgebildet.

2. Endoskoprohr nach Anspruch 1, **dadurch gekennzeichnet, daß** die Stablinsen (3) zwischen dem Randzylinder (13) und dem zweiten Stabende kegelförmig ausgebildet sind.

3. Endoskoprohr nach Anspruch 1, **dadurch gekennzeichnet, daß** der Durchmesser des Randzylinders (13) etwa einem Fünftel der Stablänge und der Durchmesser der Endfläche des zweiten Stabendes etwa einem Zehntel der Stablänge entspricht.

4. Endoskoprohr nach Anspruch 1, **dadurch gekennzeichnet, daß** die Länge des Randzylinders (13) jeweils kleiner bis gleich einem Drittel der Gesamtlänge der Stablinse (3) und größer als der halbe Durchmesser des Randzylinders (13) ist.

5. Endoskoprohr nach Anspruch 1, **dadurch gekennzeichnet, daß** die Länge des Abstandhalters (7, 7', 7"; 8; 16), der Öffnungswinkel und der Öffnungsdurchmesser der Aufnahme (10) an die Form der zweiten Stabenden derart angepaßt sind, daß bei deren Anlage in der Aufnahme (10) jeweils die Kittfläche eines Linsenelements (15) frei im Blendenraum (21) liegt und ein vorgegebener Abstand des Linsenelements (15) zur Blendenebene (19) entsteht.

6. Endoskoprohr nach Anspruch 1, **dadurch gekennzeichnet, daß** der Abstandhalter (7, 7', 7"; 8; 16) als in radialer Richtung elastischer und in axialer Richtung fester scheibenförmiger Körper (22) geformt ist, der eine in axialer Richtung durchgehende Öffnung (23) für ein Abbildungsstrahlenbündel aufweist, wobei die Außenränder der Öffnung (23) erweitert zur axial gerichteten der verjüngten Stabenden ausgebildet sind.

7. Endoskoprohr nach Anspruch 6, **dadurch gekennzeichnet, daß** die Außenränder trichterförmig erweitert zur Aufnahme konvex geformter Stabendenränder ausgebildet ist.

8. Endoskoprohr nach Anspruch 1, **dadurch gekennzeichnet, daß** der Abstandhalter (7, 7', 7"; 8; 16) aus einem elastischen Material gefertigt ist.

9. Endoskoprohr nach Anspruch 1, **dadurch gekennzeichnet, daß** der Abstandhalter (7, 7', 7"; 8; 16) am Endoskoprohr (12) radial federnd anliegt.

10. Endoskoprohr nach Anspruch 1, **dadurch gekennzeichnet, daß** der Abstandhalter (7, 7', 7"; 8; 16) einstückig als Spritzgußteil ausgeführt ist.

11. Endoskoprohr nach Anspruch 1, **dadurch gekennzeichnet, daß** der Abstandhalter (7, 7', 7"; 8; 16) aus mehreren in axialer Richtung geteilten Segmenten gefertigt ist.

## Claims

1. Endoscope tube with at least one reverse image system (5, 6) comprising two rod lenses (3), in which the first rod ends facing an intermediate image plane (18, 20) have an edge cylinder (13) mounted in the endoscope tube (12), in which the second rod ends which are positioned symmetrically with respect to a stop plane (19) have a diameter which is smaller than the internal diameter of the endoscope tube (12), wherein the end faces associated with the first rod ends are of convex design, the end faces associated with the second rod ends can be cemented together with a lens element (15) and a spacer (7, 7', 7''; 8; 16) defining the stop space (21) is inserted between the two rod lenses (3), **characterized in that** the rod diameter decreases continuously over the entire rod length in each case from the edge cylinder (13) towards the second rod end and the spacer (7, 7', 7''; 8; 16) is configured in the axial direction on both sides with a holder (10), which is matched to the reduction in the rod diameter and is designed in the shape of a funnel, for the second rod ends.

2. Endoscope tube according to Claim 1, **characterized in that** the rod lenses (3) are formed in the shape of a cone between the edge cylinder (13) and the second rod end.

3. Endoscope tube according to Claim 1, **characterized in that** the diameter of the edge cylinder (13) corresponds to about a fifth of the rod length and the diameter of the end face of the second rod end corresponds to about a tenth of the rod length.

4. Endoscope tube according to Claim 1, **characterized in that** the length of the edge cylinder (13) is in each case smaller than to equal to a third of the entire length of the rod lens (3) and larger than half the diameter of the edge cylinder (13).

5. Endoscope tube according to Claim 1, **characterized in that** the length of the spacer (7, 7', 7"; 8; 16), the opening angle and the opening diameter of the holder (10) are matched to the shape of the second rod ends such that, as they rest in the holder (10), in each case the cement area of a lens element (15) is located freely in the stop space (21) and a predetermined distance of the lens element (15) to the stop plane (19) occurs.

6. Endoscope tube according to Claim 1, **characterized in that** the spacer (7, 7', 7''; 8; 16) is formed as a body (22) which is elastic in the radial direction and is in the form of a rigid plate in the axial direction and has an opening (23), which is continuous in the axial direction, for an imaging beam bundle, wherein the external edges of the opening (23) are designed to be widened with respect to the axially directed tapered rod ends.

7. Endoscope tube according to Claim 6, **characterized in that** the external edges are designed to be widened in the form of a funnel for holding convexly shaped rod end edges.

8. Endoscope tube according to Claim 1, **characterized in that** the spacer (7, 7', 7"; 8; 16) is made of an elastic material.

9. Endoscope tube according to Claim 1, **characterized in that** the spacer (7, 7', 7"; 8; 16) rests radially resiliently on the endoscope tube (12).

10. Endoscope tube according to Claim 1, **characterized in that** the spacer (7, 7', 7"; 8; 16) is designed in one piece as injection moulding part.

11. Endoscope tube according to Claim 1, **characterized in that** the spacer (7, 7', 7"; 8; 16) is produced from a plurality of segments which are divided in the axial direction.

## Revendications

1. Tube d'endoscope comprenant au moins un système de retournement d'image (5, 6) constitué de deux lentilles barreau (3), dans lequel les premières extrémités des barreaux tournées vers un plan d'image intermédiaire (18, 20) présentent un cylindre marginal (13) qui est monté dans le tube d'endoscope (12), les deuxièmes extrémités des barreaux, situées symétriquement par rapport à un plan de diaphragme (19), présentant un diamètre qui est inférieur au diamètre intérieur du tube d'endoscope (12), les surfaces d'extrémité associées aux premières extrémités des barreaux étant réalisées sous forme convexe, les surfaces d'extrémité associées aux deuxièmes extrémités des barreaux pouvant être scellées avec un élément de lentille (15) et une entretoise (7, 7', 7" ; 8, ; 16) définissant la chambre de diaphragme (21) étant insérée entre les deux lentilles barreau (3), **caractérisé en ce que** le diamètre du barreau diminue à chaque fois en continu sur toute la longueur du barreau depuis le cylindre marginal (13) jusqu'à la deuxième extrémité du barreau et l'entretoise (7, 7', 7" ; 8 ; 16) est réalisée avec un logement (10) pour les deuxièmes extrémités des barreaux, ouvert vers la chambre de diaphragme (21), adapté à la diminution du diamètre du barreau et réalisé en forme d'entonnoir.

2. Tube d'endoscope selon la revendication 1, **caractérisé en ce que** les lentilles barreau (3) sont réalisées en forme de cône entre le cylindre marginal (13) et la deuxième extrémité du barreau.

3. Tube d'endoscope selon la revendication 1, **caractérisé en ce que** le diamètre du cylindre marginal (13) correspond approximativement à un cinquième de la longueur du barreau et le diamètre de la surface d'extrémité de la deuxième extrémité du barreau correspond approximativement à un dixième de la longueur du barreau.

4. Tube d'endoscope selon la revendication 1, **caractérisé en ce que** la longueur du cylindre marginal (13) est à chaque fois inférieure ou égale à un tiers de la longueur totale de la lentille barreau (3) et est supérieure au demi-diamètre du cylindre marginal (13).

5. Tube d'endoscope selon la revendication 1, **caractérisé en ce que** la longueur de l'entretoise (7, 7', 7" ; 8 ; 16), l'angle d'ouverture et le diamètre d'ouverture du logement (10) sont adaptés à la forme des deuxièmes extrémités des barreaux de telle sorte que lors de leur application dans le logement (10), la surface de scellement d'un élément de lentille (15) se situe à chaque fois librement dans la chambre du diaphragme (21) et qu'une distance prédéfinie de l'élément de lentille (15) par rapport au plan du diaphragme (19) soit obtenue.

6. Tube d'endoscope selon la revendication 1, **caractérisé en ce que** l'entretoise (7, 7', 7" ; 8 ; 16) est formée sous la forme d'un corps (22) élastique dans la direction radiale et en forme de disque rigide dans la direction axiale, qui présente une ouverture (23) continue dans la direction axiale pour un faisceau de rayons image, les bords extérieurs de l'ouverture (23) étant réalisés sous forme élargie par rapport aux extrémités des barreaux rétrécies orientées axialement.

7. Tube d'endoscope selon la revendication 6, **caractérisé en ce que** les bords extérieurs sont élargis en forme d'entonnoir pour recevoir des bords de forme convexe des extrémités des barreaux.

8. Tube d'endoscope selon la revendication 1, **caractérisé en ce que** l'entretoise (7, 7', 7" ; 8 ; 16) est fabriquée à partir d'un matériau élastique.

9. Tube d'endoscope selon la revendication 1, **caractérisé en ce que** l'entretoise (7, 7', 7" ; 8 ; 16) s'applique élastiquement et radialement contre le tube d'endoscope (12).

10. Tube d'endoscope selon la revendication 1, **caractérisé en ce que** l'entretoise (7, 7', 7" ; 8 ; 16) est réalisée d'une seule pièce sous forme de pièce moulée par injection.

11. Tube d'endoscope selon la revendication 1, **caractérisé en ce que** l'entretoise (7, 7', 7" ; 8 ; 16) est fabriquée à partir de plusieurs segments divisés dans la direction axiale.
